# EUROPEAN PATENT SPECIFICATION

(11) **EP 2 992 320 B1**
(45) Date of publication and mention of the grant of the patent: **12.06.2019**
(21) Application number: 14724138.4
(22) Date of filing: 30.04.2014
(51) Int. Cl.: G01N 27/333, G01N 33/18, B01J 20/26

(54) **A PHOSPHATE SENSOR, ITS USE AND ITS METHOD OF PREPARATION**
PHOSPHATSENSOR, DESSEN VERWENDUNG UND VERFAHREN ZU DESSEN HERSTELLUNG
CAPTEUR DE PHOSPHATE, SON UTILISATION ET SON PROCÉDÉ DE PRÉPARATION

(30) Priority: 30.04.2013 GB 201307777
(43) Date of publication of application: 09.03.2016
(73) Proprietor: CRANFIELD UNIVERSITY, Cranfield Bedfordshire MK43 0AL (GB); Yorkshire Water Services Limited, Yorkshire BD6 2SZ (GB)
(72) Inventor: WARWICK, Chris, Cranfield Bedfordshire MK43 0AL (GB); SOARES, Ana, Cranfield Bedfordshire MK43 0AL (GB); GUERREIRO, Antonio, Leicester Leicestershire LE1 7RH (GB)
(74) Representative: Mewburn Ellis LLP
(86) International application number: PCT/GB2014/051327
(87) International publication number: WO 2014/177856

(56) References cited:
- WO-A1-2009/064245
- US-A1- 2011 303 611
- KUGIMIYA A ET AL: "Biomimetic sensor for cAMP using an ion-sensitive field-effect transistor", MATERIALS SCIENCE AND ENGINEERING C, ELSEVIER SCIENCE S.A, CH, vol. 29, no. 3, 30 April 2009 (2009-04-30) , pages 959-962, XP026019073, ISSN: 0928-4931, DOI: 10.1016/J.MSEC.2008.09.001 [retrieved on 2008-09-07] cited in the application
- AKIMITSU KUGIMIYA ET AL: "Phosphate ion sensing using molecularly imprinted artificial polymer receptor", POLYMER BULLETIN, SPRINGER, BERLIN, DE, vol. 67, no. 9, 27 August 2011 (2011-08-27), pages 2017-2024, XP019973914, ISSN: 1436-2449, DOI: 10.1007/S00289-011-0617-6 cited in the application
- SVETLANA P. POGORELOVA ET AL: "Selective sensing of triazine herbicides in imprinted membranes using ion-sensitive field-effect transistors and microgravimetric quartz crystal microbalance measurements", THE ANALYST, vol. 127, no. 11, 8 October 2002 (2002-10-08), pages 1484-1491, XP055125188, ISSN: 0003-2654, DOI: 10.1039/b204491a
- KUGIMIYA A ET AL: "Preparation of molecularly imprinted polymers with thiourea group for phosphate", ANALYTICA CHIMICA ACTA, ELSEVIER, AMSTERDAM, NL, vol. 564, no. 2, 6 April 2006 (2006-04-06) , pages 179-183, XP027913081, ISSN: 0003-2670 [retrieved on 2006-04-06]
- XIANGYANG WU: "Molecular imprinting for anion recognition in aqueous media", MICROCHIMICA ACTA ; AN INTERNATIONAL JOURNAL ON MICRO AND TRACEANALYSIS, SPRINGER-VERLAG, VI, vol. 176, no. 1 - 2, 30 August 2011 (2011-08-30), pages 23-47, XP019997445, ISSN: 1436-5073, DOI: 10.1007/S00604-011-0683-3

## Description

### Field of the Invention

The invention concerns a phosphate sensor for detecting and measuring phosphate compounds in solution, its use and its production.

### Background

Phosphate concentrations are routinely measured in clinical diagnosis, for environmental protection, the water industry, and in various chemical industries, such as fertilizer and detergent production.

Phosphate derivatives play a significant role in biological processes and are present in a variety of key molecules including ATP, DNA, plus numerous phosphorylated proteins and phospholipids. Phosphate levels in serum are routinely measured and can aid in the diagnosis of hyperparathyroidism, vitamin D deficiency, and Fanconi syndrome (Berchmans *et al.,* 2012).

Excessive levels of phosphate in river water and wastewater effluents can result in eutrophication, leading to reduced water quality. Consequently, phosphate levels in rivers, and wastewater treatment plants are monitored by water companies and EU legislation specifies the maximum total phosphorus concentrations permitted in wastewater effluent discharges (EU Council Directive, 1991).

Phosphate based fertilizers are routinely applied in agriculture to encourage crop growth. However fertilizer applied to the soil surface is subject to agricultural run-off so that it enters rivers, lakes and reservoirs, contributing to a reduction in water quality.

The usual methods used to determine the phosphate content in environmental samples *e.g.* river and wastewater, are colorimetric techniques, developed in the 1950s. However, these methods are costly, require the addition of chemical reagents, are time consuming and create waste material, requiring safe disposal. A recent review of phosphate sensing techniques (Warwick *et al*., 2013) showed that no reliable, robust, sensing based alternative has replaced the colorimetric standard method for measuring phosphate concentrations in wastewater. Conventional electrochemical sensors suffered from limited durability, poor selectivity or both.

Molecularly imprinted polymer (MIP) particles containing thiourea based receptors have previously been prepared for the selective removal of phosphate from wastewater samples (Kugimiya and Takei, 2006) and in combination with an ion-sensitive field effect transistor (ISFET), to measure the concentration of phosphate in solution (Kugimiya and Babe, 2011). However, neither of these investigations used the thiourea receptors in a membrane format and the latter had a limited functional measurement range and did not report any possible interference from sulphate anions. Thiourea based MIP receptors have also been polymerised onto the active surface of an ISFET for use in assessing the concentration of cAMP. While the sensor showed selectivity for cAMP compared to other nucleoside monophosphates such as AMP, cGMP and GMP, the sensor was not adapted for use in detecting or measuring other molecules with phosphate based residues or phosphate ions (Kugimiya and Kohara, 2009).

US Patent No. 4,900,404 (1990) and US Patent No. 6,540,894 (2003) describe electrochemical (potentiometric) phosphate sensors based on ion selective membrane electrodes. The former has a detection range that is suitable for clinical use, but not for determining the phosphate content of environmental water. Both of the ion-selective membranes suffer from durability issues and in the latter case, the sensing membrane becomes dislodged from the transducer following prolonged exposure to harsh environments. Additionally, zinc phosphate, the functional component of the sensor membrane in the latter case, reportedly leaches out over time, inhibiting the overall functionality. Patent SU 1580233 A1 also describes an ion selective membrane based sensor, using silver/bismuth based receptors.

International Patent Application WO 2012/154028 A1 describes a potentiometric, screen printed, phosphate sensor, incorporating cobalt nanoparticles. However, cobalt based potentiometric phosphate sensors have been found to be sensitive to the amount of dissolved oxygen present in the sample, requiring simultaneous measurements of the amount of dissolved oxygen (Warwick et al., 2013). WO 2012/074368 concerns the use of a doped polypyrrole layer as conductor in a phosphate sensor. A layer is formed on top of this using a "phosphate cocktail" which is a solution including PVC and "bis-thiourea derivative ionophore", which is not further explained.

US patent 7,521,250 describes a fluorescence based optical sensor for the detection and measurement of phosphate ions and phosphorylated peptides. The sensor contains anthracene-based synthetic receptors, incorporating dipicolyamine and zinc complexes.

### Summary of the invention

This invention provides a means and method for measuring the concentration of inorganic or organic phosphate compounds in solution and a method for producing a phosphate sensor. It is particularly concerned with phosphate species that are likely to occur in water supplies, environmental water and waste water, notably orthophosphates.

The phosphate sensor comprises a solid membrane in which there are polymer-based phosphate receptors, preferably molecularly imprinted. Changes in a parameter such as conductance (or admittance) or pH are measured electrochemically and correlated with phosphate concentrations.

In one aspect the invention provides a method of making a molecularly imprinted stand-alone membrane as defined in claim 1. The template species is, or structurally resembles, a target substrate species, For example, phenylphosphonic acid may serve as a structural analogue of phosphate. Other possibilities include diphenyl phosphate and phosphoric acid (e.g. H₃PO₄).

Typically, one or more polymerisable monomers (functional monomers) and a mixture of cross-linking agents are polymerised in the presence of a template species, in conditions such that the polymer is produced in the form of a stand-alone membrane. The polymer is then treated to remove the template species. This leaves sites with affinity for phosphate species. The formation of the membrane involves monomers that, when polymerised, can have affinity to phosphate compounds. A preferred class is thiourea species, e.g. N-allylthiourea or other thioureas having polymerisable (e.g. ethylenically unsaturated) groups attached to one or both nitrogen atoms. Another class of monomer is transition metal compounds with polymerisable moieties, e.g. (meth)acrylate salts.

In another aspect the invention provides a phosphate sensor incorporating a phosphate sensor membrane produced by the method according to the first aspect. A phophate sensing apparatus also includes a cell for receiving an analyte solution, a pair of spaced electrodes in the cell, and the polymer-membrane between the electrodes. The electrodes are connected to a meter for measuring resistance or conductance (or admittance).

In a third aspect the invention provides a method for detecting and/or measuring the concentration of one or more target phosphate species in an analyte solution using the phosphate sensing apparatus according to claim 7 or claim 8.

Usually, the sensor's recognition element comprises a solid, molecularly imprinted, polymer membrane, produced using (a) a template molecule that displays structural similarity to a phosphate anion, (b) functional monomers such as vinyl monomers, allyl monomers, acetylenes, methacrylates, acrylates, methacrylic and acrylic derivatives of thioureas or of transition metals and their salts *e.g.* titanium, zirconium and iron; other possibilities include derivatives of amino acids, nucleosides, nucleotides and carbohydrates (derivativised to provide polymerisable moieties) and (c) cross-linking monomers which serve to fix, or stabilise, the structure of the resulting replica molecule, so that it remains complementary to that of the template.

Functional monomers are thioureas with unsaturated moieties, particularly N-allylthiourea and N-methyl-N(4-vinylphenyl)thiourea. Also favoured are zirconium carboxyethyl acrylate and other acrylic derivatives of transition metals including zirconium acrylate, zirconium methacrylate, hafnium acrylate and iron acrylate.

Typical examples of cross-linkers suitable for MIPs include ethylene glycol dimethacrylate, methylene bisacrylamide, N,N'-bisacryloylpiperazine and oligourethane acrylate. The function of cross-linking agents can be performed by particles containing double bonds, or particles with multiple functionalities attached which can bind to functional monomers. Those skilled in the art could select monomers and cross-linkers suitable for a particular system. Alternatively, a variety of combinatorial and computational methods could be used to assist in this selection.

In one type of embodiment, the sensor response is not potentiometric. Rather, on binding inorganic or organic phosphate compounds, there is a measureable change in conductance (resistance, impedance, or admittance) in proportion to the concentration of phosphate present in solution. The changes in conductance, resistance, impedance or admittance, are generated due to conformational changes in the polymer structure and ionic interactions resulting from the phosphate in test samples binding to the sites with affinity, usually imprinted cavities formed by a template molecule during polymerisation.

The change in conductance (resistance, impedance, or admittance) is preferably manifested in a solid membrane.

The specific combination of phenylphosphonic acid and thiourea based MIPs produced in a membrane format, with conductance based transduction, has been found to provide a more robust, convenient and reliable approach to phosphate measurement than the current practices in the water industry. Current methods are colorimetric and time consuming. Furthermore, they require the use of additional reagents and materials, creating waste, which needs safe disposal, adding additional costs. Having a reusable sensor that can directly measure the concentration of phosphate can provide a cost effective and easy to use alternative.

Conductance measurements are frequently used in environmental monitoring to examine water quality and record total salt concentrations at wastewater treatment plants. However, the presence of any ion in solution will produce a change in conductance. By incorporating a receptor, our system offers specificity enabling the measurement of a particular ion. Use of an embedded MIP, thiourea based, phosphate receptor allows a change in conductance when the membrane binds charged phosphate ions (but not other ions), providing the required selectivity.

Potentiometric sensors are often used to measure ions in solution, so a good deal of research has focussed on the creation of a potentiometric based phosphate sensor. However, these methods have largely failed, as they either show poor selectivity for phosphate or have limited durability when used in the field. However our type of membrane can also be used in a potentiometric sensor, retaining our advantages of selectivity and durability. For example, the tip of a pH electrode can receive, or be replaced by, a layer of our membrane. When the pH electrode and a reference electrode are immersed in a test solution, the potential difference between them is correlated with the concentration of phosphate in the test solution.

### Brief Description of Drawings

Some embodiments of the invention will now be described with reference to the accompanying drawings in which:
Fig. 1 is a schematic view of a cell comprising a first sensor embodying the invention.
Fig. 2 is calibration curve showing sensor response using wastewater samples with different phosphate concentrations.
Fig. 3 is a plot showing sensor response on applying equimolar solutions of phosphate, sulphate and nitrate at time t=0.
Fig. 4 is a schematic view of a cell comprising a second sensor embodying the invention.

### Detailed description of the embodiments

The procedure for manufacturing a first embodiment of the sensor is as follows.

### Outline

A receptor membrane is prepared by combining a template molecule, which comprises a residue which is a structural analogue of phosphate; one or more functional monomers, such as vinyl monomers, allyl monomers, acetylenes, acrylates, methacrylates, or derivatives of amino acids, nucleosides, nucleotides and carbohydrates; a mixture of cross linking reagents to provide a combination of rigidity and flexibility, for example, ethylene glycol dimethacrylate, methylene bisacrylamide or N,N'-bisacryloylpiperazine; together with a solvent, for example dimethylformamide (DMF) or acetonitrile (ACN) and a polymerisation initiator, preferably azobis-cyclohexanecarbonitrile. The polymerisation mixture is polymerised, suitably under UV. The template is then removed from the polymer membrane.

A section of the receptor membrane is positioned between two, or more, electrodes, positioned a fixed distance apart, on either side of the membrane, and connected to a measuring device, such as an ohmmeter. The phosphate sensor is ready to be used and the change in conductance (resistance, impedance or admittance) is recorded on placing in the sensor in a test solution containing phosphate.

### Details

### (i) Preparation of receptor membrane

The receptor membrane was prepared by combining a mixture of cross linking reagents (ethylene glycol dimethacrylate (EGDMA) and oligourethane acrylate (OUA) in ratios ranging from 8:1 to 1:1) together with a functional monomer, N-allylthiourea, in a molar ratio (of crosslinkers to monomer) ranging from 8:1 to 1:1.A structural analogue of phosphate, phenylphosphonic acid, was used as a template molecule and added to the polymerisation mixture in ratios (of monomer to template) ranging from 8:1 to 1:1. Dimethylformamide (DMF) solvent was added, together with a polymerisation initiator, azobis-cyclohexanecarbonitrile. The polymerisation mixture was inserted between two glass plates with a silicone perimeter and polymerised under UV. The polymer membrane was then cleansed (removing reagents, by-products and the template) using overnight Soxhlet extraction with methanol.

The favoured composition was : EGDMA : OUA = 6:1; crosslinkers : monomer = 4 : 1; functional monomer : template = 2 : 1.

### (ii) Integration of receptor membrane into conductance cell based sensor

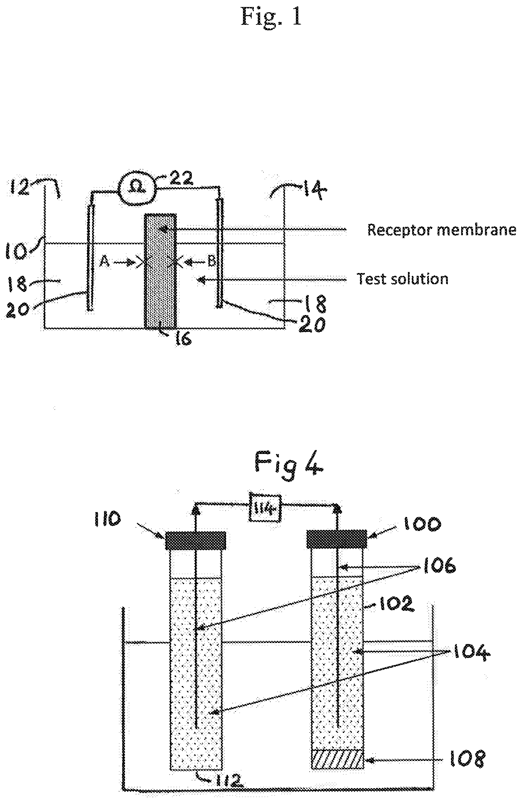

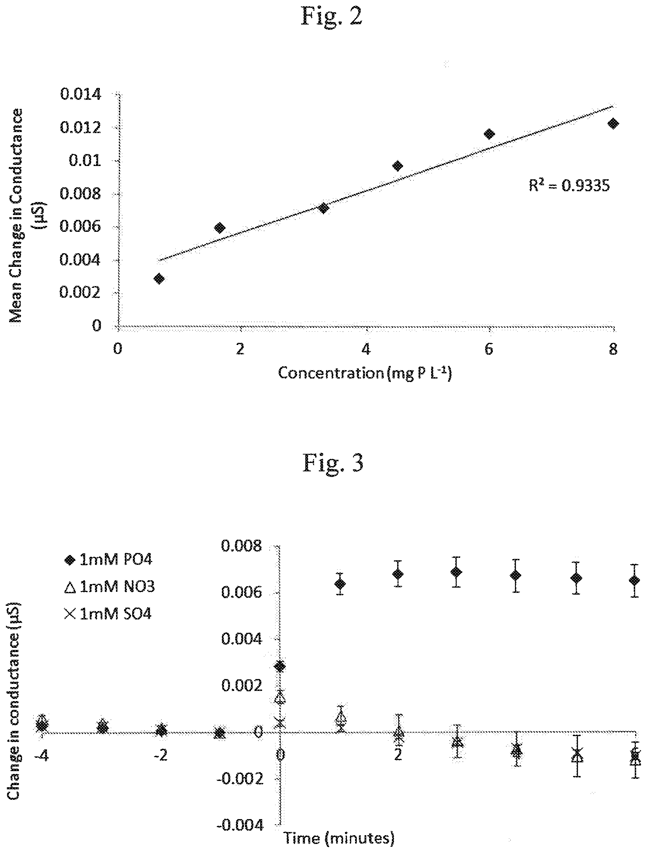

A section of the receptor membrane was positioned between two interconnecting wells in a sensor cell 10, creating two sample wells 12,14 separated by the membrane 16 (Figure 1). A chloride based electrolyte solution 18 was added to each well and electrodes 20, preferably platinum, were positioned a fixed distance apart in each well, on either side of the membrane, and connected to an ohmmeter 22. Changes in conductance were recorded on the addition to the sample wells of laboratory or wastewater test samples, which contained varying concentrations of soluble phosphate anions, producing the calibration curves shown in Figure 2. Repeated use of the sensor was achieved by rinsing the membrane with deionised water.

Additional tests to show the selectivity for phosphate compared to other common oxyanions (sulphate and nitrate) produced the results shown in Figure 3.

Fig. 4 shows a variant that is based on a pH meter. It uses the same thiourea based MIP receptor, in membrane format as the first embodiment. However this membrane is reinforced with glass fibre and attached to the tip of a pH glass electrode (e.g. silver chloride). The method for attaching a polymer membrane prepared by UV initiated free-radical polymerisation to a glass surface using adhesion promoters is documented elsewhere (Lin, W.C., Yang, C.H., Wang, T.L., Shieh, Y.T. and Chen, W.J. Express Polymer Letter, 2012, (6) pp 2-13) and summarised below.

The tip of a glass pH electrode is cleansed with chloroform and sodium hydroxide in order to activate the glass surface and then undergoes a silanization process with an adhesion promoter (using 3-(trimethoxysilyl)propyl methacrylate, which comprises a silane group in conjunction with a methacrylate group) to facilitate the attachment of the MIP membrane to the glass surface.

Thiourea based MIP membranes, reinforced with glass fibre, are prepared by combining N-allylthiourea EGDMA, phenylphosphonic acid, DMF and azobis-cyclohexanecarbonitrile) and positioning the solution on the silanized pH glass probe tip and polymerising with UV light. The pH probe tips, with the attached MIP membrane, are then rinsed in methanol in order to remove the phenylphosphonic acid template.

The result is a phosphate-sensitive glass electrode 100 which comprises a glass tube 102 containing a chloride-based internal electrolyte 104 and a silver internal electrode 106. The bottom end of the tube is closed by the MIP membrane 108.

A reference electrode 110 is almost identical, but its bottom end is closed by a simple ion-permeable membrane 112.

To perform measurements, the modified pH MIP electrode 100 and the reference electrode 112 (e.g. silver chloride) are immersed in a test solution and the pH (or potential difference) recorded on a pH meter 114. This pH (potential difference) is then compared to the baseline pH (potential difference) of the test solution. The change in pH is proportional to the concentration of phosphate in the test solution.

### References:

Berchmans, S., Issa, T. and Singh, P. (2012). Determination of inorganic phosphate by electroanalytical methods: A review. Analytica Chimica Acta, 729, 7-20.
EU Council Directive. (1991, May 21). *Council Directive concerning urban waste water treatment.* Retrieved Dec 12, 2009, from Urban waste water treatment: http://eur-lex.europa.eu/LexUriServ/site/en/consleg/1991/L/01991L0271-19980327-en.pdf International Patent Application WO 2012/154028 A1 (2012).
International Patent Application WO 2012/074368.
Kugimiya, A. and Babe, F. (2011). Phosphate ion sensing using molecularly imprinted artificial polymer receptors. Polymer Bulletin, 67, 2017-2024.
Kugimiya, A. and Kohara, K. (2009). Biomimetic sensor for cAMP using an ion-sensitive field-effect transistor. Materials Science and Engineering C, 29, 959-962.
Kugimiya, A. and Takei, H. (2006). Preparation of molecularly imprinted polymers with thiourea group for phosphate. Analytica Chimica Acta, 564, 179-183.
Patent number SU 1580233 A1 (1987).
US Patent No. 6,540,894 (2003).
US Patent No. 4,900,404 (1990).
US Patent No. 7,521,250 B2 (2009).
Warwick, C., Guerreiro, A. and Soares, A. (2013). Sensing and Analysis of Soluble Phosphates in Environmental Samples: A Review. Biosensors and Bioelectronics, 41, 1-11.

## Claims

1. A method of making a molecularly imprinted stand-alone membrane for use in a phosphate sensor, the method comprising polymerising one or more polymerisable components comprising a thiourea species having at least one polymerisable moiety attached to at least one of its nitrogen atoms, said polymerisable components also including a mixture of cross-linking agents, and said polymerisation taking place in the presence of a template compound that is or structurally resembles a target phosphate species.

2. A method according to claim 1 wherein the template compound is selected from phenylphosphonic acid, diphenyl phosphate and phosphoric acid.

3. A method according to claim 1 wherein the cross-linking agents comprise one or more of ethylene glycol dimethacrylate, methylene bisacrylamide, N,N'-bisacryloylpiperazine and oligourethane acrylate.

4. A method according to any of claims 1 to 3 including a further step of treating the membrane to remove template compound.

5. A method according to any of claims 1 to 4 wherein the thiourea species is N-allylthiourea.

6. A phosphate sensor incorporating a phosphate sensor membrane as produced by the method of any of claims 1 to 5,

7. Phosphate sensing apparatus comprising a vessel (10) for holding a test solution, said vessel being partitioned into two electrode compartments (12, 14) by a partition comprising a phosphate sensor membrane (16) as produced by the method of any of claims 1 to 5, and further including respective electrodes (20) extending into the two compartments and a meter (22) connected to the electrodes for measuring resistance or conductance.

8. Phosphate sensing apparatus comprising a pH probe electrode (100) modified to include a phosphate sensor membrane (108) as produced by the method of any of claims 1 to 5, and further including a vessel for holding a test solution, a reference electrode (110), and a meter (114) connected to the two electrodes (100, 112) for measuring potential differences.

9. A method of analysing a sample for phosphate by using apparatus according to claim 7 or claim 8.

## Patentansprüche

1. Verfahren zur Herstellung einer molekular bedruckten selbsttragenden Membran zur Verwendung in einem Phosphatsensor, wobei das Verfahren das Polymerisieren einer oder mehrerer polymerisierbarer Komponenten umfasst, die eine Thioharnstoff-Spezies mit zumindest einer an zumindest eines ihrer Stickstoffatome gebundenen polymerisierbaren Gruppierung umfassen, wobei die polymerisierbaren Komponenten auch ein Gemisch von Vernetzungsmitteln umfassen und die Polymerisation in Gegenwart einer Matrizenverbindung stattfindet, die einer Ziel-Phosphatspezies strukturell ähnlich ist.

2. Verfahren nach Anspruch 1, wobei die Matrizenverbindung aus Phenylphosphonsäure, Diphenylphosphat und Phosphorsäure ausgewählt ist.

3. Verfahren nach Anspruch 1, wobei die Vernetzungsmittel eines oder mehrere von Ethylenglykoldimethacrylat, Methylenbisacrylamid, N,N'-Bisacryloylpiperazin und Oligourethanacrylat sind.

4. Verfahren nach einem der Ansprüche 1 bis 3, das einen weiteren Schritt der Behandlung der Membran zur Entfernung von Matrizenverbindung umfasst.

5. Verfahren nach einem der Ansprüche 1 bis 4, wobei die Thioharnstoff-Spezies N-Allylthioharnstoff ist.

6. Phosphatsensor, der eine durch ein Verfahren nach einem der Ansprüche 1 bis 5 hergestellte Phosphatsensormembran umfasst.

7. Phosphatdetektionsvorrichtung, die ein Gefäß (10) zur Aufnahme einer Testlösung umfasst, wobei das Gefäß durch eine Teilung, die eine durch ein Verfahren nach einem der Ansprüche 1 bis 5 hergestellte Phosphatsensormembran (16) umfasst, in zwei Elektrodenkammern (12, 14) unterteilt ist, und die außerdem entsprechende Elektroden (20), die sich in die beiden Kammern erstrecken, sowie ein mit den Elektroden verbundenes Messgerät (22) zum Messen von Widerstand oder Leifähigkeit umfasst.

8. Phosphatdetektionsvorrichtung, die eine pH-Sondenelektrode (100), die so modifiziert ist, dass sie eine durch ein Verfahren nach einem der Ansprüche 1 bis 5 hergestellte Phosphatsensormembran (108) umfasst, und außerdem ein Gefäß zur Aufnahme einer Testlösung, eine Referenzelektrode (110) und ein mit den zwei Elektroden (100, 112) verbundenes Messgerät (114) zum Messen von Potentialdifferenzen umfasst.

9. Verfahren zum Analysieren einer Probe in Bezug auf Phosphat unter Verwendung einer Vorrichtung nach Anspruch 7 oder 8.

## Revendications

1. Procédé de fabrication d'une membrane autonome à empreinte moléculaire destinée à être utilisée dans un capteur de phosphate, le procédé comprenant la polymérisation d'un ou plusieurs composants polymérisables comprenant une espèce de thio-urée ayant au moins un fragment polymérisable lié à au moins l'un de ses atomes d'azote, lesdits composants polymérisables comprenant également un mélange d'agents de réticulation, et ladite polymérisation ayant lieu en présence d'un composé matrice qui est ou ressemble structurellement à une espèce de phosphate cible.

2. Procédé selon la revendication 1, dans lequel le composé matrice est choisi parmi l'acide phénylphosphonique, le phosphate de diphényle et l'acide phosphorique.

3. Procédé selon la revendication 1, dans lequel les agents de réticulation comprennent un ou plusieurs parmi le diméthacrylate d'éthylène glycol, le bisacrylamide de méthylène, la N,N'-bisacryloylpipérazine et l'acrylate d'oligo-uréthane.

4. Procédé selon l'une quelconque des revendications 1 à 3, comprenant une étape supplémentaire de traitement de la membrane pour éliminer le composé matrice.

5. Procédé selon l'une quelconque des revendications 1 à 4, dans lequel l'espèce de thio-urée est la N-allylthio-urée.

6. Capteur de phosphate incorporant une membrane de capteur de phosphate telle que produite par le procédé selon l'une quelconque des revendications 1 à 5.

7. Appareil de détection de phosphate comprenant un récipient (10) destiné à contenir une solution de test, ledit récipient étant divisé en deux compartiments d'électrode (12, 14) par une cloison comprenant une membrane de capteur de phosphate (16) telle que produite par le procédé selon l'une quelconque des revendications 1 à 5, et comprenant en outre des électrodes respectives (20) s'étendant dans les deux compartiments et un compteur (22) connecté aux électrodes pour mesurer la résistance ou la conductance.

8. Appareil de détection de phosphate comprenant une électrode de sonde de pH (100) modifiée pour inclure une membrane de capteur de phosphate (108) telle que produite par le procédé selon l'une quelconque des revendications 1 à 5, et comprenant en outre un récipient destiné à contenir une solution de test, une électrode de référence (110) et un compteur (114) connecté aux deux électrodes (100, 112) pour mesurer des différences de potentiel.

9. Procédé d'analyse d'un échantillon pour le phosphate en utilisant un appareil selon la revendication 7 ou la revendication 8.
